# EUROPÄISCHE PATENTANMELDUNG

(11) **EP 4 831 997 A1**
(43) Veröffentlichungstag der Anmeldung: **28.10.2026**
(21) Anmeldenummer: 25172695.6
(22) Anmeldetag: 25.04.2025
(51) Int. Cl.: G16H 10/60, G16H 20/10, G16H 20/70, G16H 50/20, G16H 50/70, G06N 3/006

(54) **COMPUTER-IMPLEMENTIERTES VERFAHREN ZUM ASSISTIEREN MENSCHLICHEN VERHALTENS**

(71) Anmelder: CU Innovations UG, 65779 Kelkheim Taunus (DE)
(72) Erfinder: Kulens, Can, 65779 Kelkheim Taunus (DE); Erguen, Ufuk, 65779 Kelkheim Taunus (DE)
(74) Vertreter: WSL Patentanwälte Partnerschaft mbB

(57) **Zusammenfassung**

Die vorliegende Erfindung betrifft ein computer-implementiertes Arbeitsverfahren zum Erzeugen einer mit menschlichen Sinnesorgangen wahrnehmbaren Nachricht für eine erste menschliche Person sowie ein computer-implementiertes Verfahren zum Spezifizieren einer künstlichen Intelligenz. Beide Verfahren ermöglichen oder sind ein computer-implementiertes Verfahren zum Assistieren menschlichen Verhaltens. Das Arbeitsverfahren beschreibt die Anwendung der künstlichen Intelligenz, das Trainingsverfahren dessen Herstellung.

## Beschreibung

Die vorliegende Erfindung betrifft ein computer-implementiertes Arbeitsverfahren zum Erzeugen einer mit menschlichen Sinnesorgangen wahrnehmbaren Nachricht für eine erste menschliche Person sowie ein computer-implementiertes Verfahren zum Spezifizieren einer künstlichen Intelligenz. Beide Verfahren ermöglichen oder sind ein computer-implementiertes Verfahren zum Assistieren menschlichen Verhaltens. Das Arbeitsverfahren beschreibt die Anwendung der künstlichen Intelligenz, das Trainingsverfahren dessen Herstellung.

Die medizinische Versorgung steht in vielen Gesundheitssystemen weltweit vor einer zunehmenden Komplexität, insbesondere im Hinblick auf die Diagnostik und Therapie von Patientinnen und Patienten. Trotz des technischen Fortschritts und der zunehmenden Verfügbarkeit digitaler Hilfsmittel bleibt die Informationslage für behandelnde Ärztinnen und Ärzte häufig lückenhaft oder fragmentiert. In der klinischen Praxis bedeutet dies, dass medizinisches Fachpersonal oftmals nur einen eingeschränkten Einblick in die vollständige Patientenhistorie erhält. Dies gilt insbesondere dann, wenn Patientinnen und Patienten bei unterschiedlichen Leistungserbringern behandelt wurden oder wenn eine sektorenübergreifende Versorgung stattfindet, etwa zwischen ambulanter und stationärer Behandlung.

Diese unzureichende Informationsverfügbarkeit kann direkte Auswirkungen auf die Qualität medizinischer Entscheidungen haben. Die Folge sind potenziell vermeidbare Fehldiagnosen, suboptimale Therapieentscheidungen und im Extremfall patientenschädigende Maßnahmen. Diese Problematik wird weiter verschärft durch die Tatsache, dass medizinische Entscheidungen nicht isoliert getroffen werden, sondern oftmals im Spannungsfeld knapper Zeitressourcen und hoher Arbeitsbelastung erfolgen müssen. Gerade in solchen Kontexten ist ein umfassendes, präzises und schnell zugängliches Wissen über den individuellen Patienten von zentraler Bedeutung.

Ein wesentliches Hindernis für ein modernes und effektives Wissensmanagement im medizinischen Bereich liegt in der weiterhin weit verbreiteten Nutzung veralteter Kommunikationsmittel. So ist beispielsweise der Einsatz von Faxgeräten zur Übermittlung von medizinischen Dokumenten - darunter Befunde, Überweisungen und Arztbriefe - auch im digitalen Zeitalter noch gängige Praxis in vielen Gesundheitseinrichtungen. Die dadurch bedingte manuelle Weiterverarbeitung, fehlende Strukturierung der Daten sowie die eingeschränkte Interoperabilität zwischen IT-Systemen behindern einen schnellen, sicheren und datenschutzkonformen Informationsaustausch. Selbst wenn elektronische Patientenakten existieren, sind sie häufig nicht vollständig, nicht aktuell oder nicht systemübergreifend abrufbar.

Die Problematik wird durch die besondere Sensibilität medizinischer Daten zusätzlich verschärft. Strenge Datenschutzvorschriften - wie etwa die europäische Datenschutzgrundverordnung (DSGVO) - sind einerseits unabdingbar zum Schutz der Persönlichkeitsrechte der Patienten, stellen andererseits jedoch auch hohe Anforderungen an Systeme zur Datenspeicherung, -verarbeitung und -weitergabe. Viele Gesundheitseinrichtungen sehen sich daher in einem Spannungsfeld zwischen regulatorischen Anforderungen und dem praktischen Bedarf nach effizientem Informationsaustausch.

Ein weiterer Aspekt, der die bestehende Problemlage verschärft, ist die Diskrepanz zwischen medizinischer Forschung und klinischer Praxis. Während die Forschung in der Medizin ein hohes Innovationstempo aufweist und kontinuierlich neue Erkenntnisse etwa zu Diagnostikmethoden, Therapieansätzen oder Risikofaktoren publiziert werden, gelangen diese wissenschaftlichen Fortschritte häufig nur mit erheblicher Verzögerung in den klinischen Alltag. Die Gründe hierfür sind vielfältig und reichen von fehlenden strukturellen Schnittstellen zwischen Forschungseinrichtungen und Versorgungspraxen bis hin zu mangelnden Ressourcen für die Umsetzung neuer Erkenntnisse in standardisierte Leitlinien und Versorgungsprozesse.

Zusammengenommen ergibt sich somit ein vielschichtiges Problemfeld im Bereich des medizinischen Wissens- und Informationsmanagements: fragmentierte Patienteninformationen, intransparente Kommunikationswege, hohe Datenschutzanforderungen und eine verzögerte Translation medizinischer Innovationen in den Versorgungsalltag. Diese strukturellen Defizite wirken sich nicht nur negativ auf die Behandlungsqualität aus, sondern hemmen auch die Effizienz des gesamten Gesundheitssystems.

Vor diesem Hintergrund besteht die Aufgabe der vorliegenden Erfindung darin, eine ganzheitliche Sicht auf den Patienten oder sich selbst zu ermöglichen sowie den sicheren und strukturierten Austausch medizinischer Informationen zu ermöglichen, um somit schnelle und effiziente medizinische Entscheidungen für andere oder sich selbst treffen zu können.

Diese Aufgabe wird gelöst durch ein computer-implementiertes Arbeitsverfahren zum Erzeugen einer mit menschlichen Sinnesorgangen wahrnehmbaren Nachricht für eine erste menschliche Person. Das Verfahren umfasst dabei folgende Schritte:
A) Dauerhaftes oder wiederholtes Erhalten von mindestens einer zeitlich veränderlichen und mit einem Mobilgerät messbaren digitalen Umgebungsinformation der ersten menschlichen Person und/oder der zweiten Person wie zum Beispiel geobasierten Informationen (GPS-Signale, WLAN -Standorte) oder einer digitalen akustischen Information in Form einer digitalen Sprach- und/oder Tonaufnahme eines Mikrofons,
   und/oder
   dauerhaftes oder wiederholtes Erhalten von mindestens einer digitalen zeitlich veränderlichen Zustandsinformation einer zweiten menschlichen Person wie zum Beispiel der gemessenen Herzfrequenz der zweiten menschlichen Person;
B) Erhalten von mindestens einer digitalen historischen Gesundheitsinformation der zweiten menschlichen Person, wie zum Beispiel dem Impfstatus der zweiten menschlichen Person oder dem aktuellen Medikationsplan der zweiten menschlichen Person, aus einer ersten Computerdatenbank;
C) Bestimmen eines digitalen medizinischen Analyseergebnisses, insbesondere eines Anamneseergebnisses der zweiten menschlichen Person und/oder einer medizinischen Diagnose der zweiten menschlichen Person und/oder eines medizinischen Verhaltensvorschlags für die zweite menschliche Person und/oder eines medizinischen Therapievorschlag für die zweite menschliche Person,
   - in Abhängigkeit von der in Schritt A erhaltenen Umgebungsinformation, falls in SchrittA eine Umgebungsinformation erhalten worden ist, und/oder in Abhängigkeit von der in Schritt A erhaltenen Zustandsinformation, falls in Schritt A eine Zustandsinformation erhalten worden ist, und
   - in Abhängigkeit von der in Schritt C erhaltenen historischen Gesundheitsinformation und
   - unter Verwendung einer trainierten künstlichen Intelligenz,
   wobei die künstliche Intelligenz derart trainiert ist, dass sie beim Erhalt mindestens zweier Eingangsgrößen, nämlich der digitalen Umgebungsinformation oder der digitalen Zustandsinformation einerseits und der digitalen Gesundheitsinformation andererseits, ein digitales medizinisches Analyseergebnis als Ausgangsgröße ausgibt;
D) Erzeugen der wahrnehmbaren Nachricht, wobei die wahrnehmbare Nachricht derart ausgebildet ist, dass die wahrnehmbare Nachricht das medizinische Analyseergebnis für die erste menschliche Person erfassbar macht.

Die Nachricht kann zusätzlich in vereinfachter oder mehrsprachiger Form zugänglich bereitgestellt werden. Sie kann visuell, akustisch oder haptisch wahrnehmbar gemacht werden, beispielsweise über ein Display, einen Kopfhörer oder ein Vibrationssignal.

Die erste menschliche Person kann zum Beispiel eine Assistenzärztin sein und die zweite menschliche Person kann in diesem Beispiel eine von der Assistenzärztin behandelte Patientin sein. Eine Umgebungsinformation der Assistenzärztin kann beispielsweise eine dauerhaft erfasste Audioaufnahme, insbesondere Sprachaufnahme, sein, die mit einem Mikrofon, welches die Assistenzärztin bei sich trägt, erfasst wird. So kann als Umgebungsinformation zum Beispiel ein Anamnesegespräch mit der zweiten menschlichen Person erfasst werden. Tritt dann eine Gesundheitsinformation der Patientin hinzu, zum Beispiel der aktuelle Medikamentenplan der Patientin, so erzeugt die trainierte künstliche Intelligenz basierend auf dem Anamnesegespräch ein Analyseergebnis des Gesprächs und erkennt, ob und wann im Gespräch eine Intervention nötig sein könnte, um das Gespräch in eine effizientere Richtung zu lenken - beispielsweise, weil die gesprochenen Angaben der Patientin zu ihrer Medikation nicht mit dem hinterlegten Medikamentenplan zusammenpassen. Dann wird im Schritt D eine für die Assistenzärztin wahrnehmbare Nachricht erzeugt, zum Beispiel in Form einer durch eine Augmented-Reality-Brille lesbaren Nachricht, mit der die Assistentsärztin über diese Inkonsistenz zwischen Gesagtem und Hinterlegtem informiert wird.

Insbesondere wird im Schritt A eine digitale Umgebungsinformation der ersten menschlichen Person erfasst. Dies ermöglicht in einem der genannten Beispiele, dass die Umgebungsinformation an die behandelnde medizinische Fachkraft gekoppelt ist.

In einem anderen Beispiel sind die erste menschliche Person und die zweite menschliche Person ein und dieselbe menschliche Person. Das erfindungsgemäße Verfahren kann dann zur Selbstoptimierung und Gesundheitsprävention genutzt werden. Wird zum Beispiel die geographische Position der Person als Umgebungsinformation erhalten und gleichzeitig der Impfstatus der Person als Gesundheitsinformation, so kann eine Analyse erfolgen, mit welchem Risiko sich die Person einer Infektion mit einer Infektionskrankheit aussetzt und dies als digitales medizinisches Analyseergebnis präventiv der Person in Form der Nachricht (in Schritt D) zu Verfügung gestellt werden.

Der Begriff "medizinisch" bezieht sich im Sinne der vorliegenden Erfindung auf alles, was mit der Identifikation, Diagnose, Bewertung, Überwachung, Behandlung oder Prävention von physischen und psychischen Zuständen, Erkrankungen oder Störungen in Zusammenhang steht. Dies schließt Methoden, Techniken und Prozesse ein, die verwendet werden, um physische sowie psychische Gesundheitsinformationen zu analysieren und zu interpretieren. "Medizinische" Analyseergebnisse umfassen daher sowohl physiologische Daten, die sich auf die körperliche Gesundheit beziehen, als auch psychologische Daten, die emotionale, kognitive und verhaltensbezogene Aspekte einer Person betreffen. Der Begriff bezieht sich auf die Nutzung jeglicher Art von Geräten, Systemen oder Verfahren, die in der Lage sind, solche Daten zu erfassen, zu verarbeiten oder zu analysieren, unabhängig davon, ob diese für klinische oder nicht-klinische Zwecke eingesetzt werden.

Eine wahrnehmbare Nachricht ist im Sinne der vorliegenden Erfindung insbesondere eine akustische, visuelle oder haptisch wahrnehmbare Nachricht. Eine "Nachricht" im technischen Sinn ist jede Art von Information oder Daten, die in einer für die Übertragung, Verarbeitung oder Speicherung geeigneten Form kodiert oder konfiguriert sind. Dies umfasst digitale und analoge Daten, die in jeglicher Form von Signalen, Symbolen, Schriftzeichen oder anderen Mitteln ausgedrückt werden können. Die Nachricht kann über beliebige Medien übertragen werden, einschließlich, aber nicht beschränkt auf elektrische, optische, akustische oder drahtlose Übertragungskanäle. Dabei kann die Nachricht verschiedene Formate haben, wie zum Beispiel Text, Audio, Video, Bild oder jede Kombination dieser Elemente. Die Nachricht kann insbesondere als standardisierter Arztbrief ausgebildet sein, der zum Beispiel auf der Basis mehrerer in Schritt B erfasster Gesundheitsinformationen, die anschließend in Schritt C gemeinsam von der künstlichen Intelligenz analysiert werden, erstellt wird.

Der Begriff "Umgebungsinformation" bezeichnet im Sinne der vorliegenden Erfindung eine Information, die Merkmale, Zustände oder Eigenschaften der physischen, chemischen, biologischen, sozialen oder technologischen Umgebung einer menschlichen Person beschreibt oder abbildet. Sie kann zeitlich und räumlich variabel sein und umfasst beispielsweise Daten zu Temperatur, Luftqualität, Lichtverhältnissen, Geräuschpegel, sprachlichen Äußerungen (z. B. gesprochene Sprache, gesprochene Inhalte oder Sprachmuster), elektromagnetischen Feldern, geographischer Position, Anwesenheit und Verhalten anderer Personen, Umgebungsobjekten sowie zu räumlichen oder situativen Gegebenheiten. Umgebungsinformationen können durch Sensoren, Mikrofone, manuelle Eingaben, externe Datenquellen oder andere technische Mittel erfasst werden. Sie dienen dann in Schritt C der Auswertung im Zusammenhang mit medizinischen, gesundheitsbezogenen oder verhaltensanalytischen Fragestellungen Gemäß einer Ausführungsform des erfindungsgemäßen Arbeitsverfahrens werden mehrere Umgebungsinformationen der ersten menschlichen Person in Schritt A erfasst, wobei in Schritt C das medizinische Analyseergebnis in Abhängigkeit dieser mehreren Umgebungsinformationen bestimmt wird. Dies ermöglicht eine höhere Genauigkeit bei der Bestimmung des medizinischen Analyseergebnisses.

Der Begriff "Zustandsinformation" bezeichnet im Sinne der vorliegenden Erfindung eine Information, die Merkmale, Parameter oder Eigenschaften einer bestimmten menschlichen Person beschreibt oder erfasst, welche den aktuellen physischen, physiologischen, psychischen oder verhaltensbezogenen Zustand dieser Person betreffen. Zustandsinformationen können zeitlich variabel sein und umfassen beispielsweise Werte wie Körpertemperatur, Herzfrequenz, Atemfrequenz, Blutdruck, Hautleitfähigkeit, Hormonspiegel, Schlafmuster, Bewegungsmuster, Aktivitätsniveau, emotionale Indikatoren, kognitive Parameter oder subjektiv angegebene Zustände. Die Erhebung kann automatisiert (z. B. durch Sensoren oder Messgeräte), manuell oder durch Auswertung anderer Datenquellen erfolgen.

Zur Erhebung von Zustandsinformationen im Sinne der vorliegenden Anmeldung können tragbare elektronische Geräte (sogenannte Wearables) eingesetzt werden, die mit geeigneten Sensoren zur Erfassung physiologischer, physischer, psychischer oder verhaltensbezogener Parameter ausgestattet sind. Solche Wearables umfassen insbesondere Smartwatches, Fitnessarmbänder, smarte Ringe, tragbare EEG- oder EKG-Geräte, textile Sensorplattformen (z. B. integrierte Sensorik in Kleidung), Brillen mit integrierten Messsystemen sowie an der Haut angebrachte Patches oder Pflaster. Zustandsinformationen können sich sowohl auf unmittelbar gemessene Messwerte als auch auf daraus abgeleitete bzw. approximative Parameter stützen, die mithilfe von Datenfusion, statistischen Schätzungen oder Modellberechnungen gewonnen werden.

Diese Geräte können mit Sensoren wie Photoplethysmographie-Sensoren (PPG), Elektroden zur bioelektrischen Impedanzanalyse, elektrodermalen Sensoren, Beschleunigungs- und Gyroskopsensoren, optischen Sensoren zur Messung der Hautdurchblutung, Temperaturfühlern, Mikrofonen, Hautspannungssensoren oder chemischen Sensoren zur Analyse von Schweiß oder anderen Körperausscheidungen ausgestattet sein. Durch Kombination mehrerer Sensorarten innerhalb eines Wearables ist eine multimodale Erfassung verschiedener Zustandsinformationen in Echtzeit möglich.

Die über Wearables gewonnenen Messwerte können kontinuierlich oder intervallweise erfasst werden.

Gemäß einer Ausführungsform des erfindungsgemäßen Arbeitsverfahrens werden mehrere Zustandsinformationen der zweiten menschlichen Person in Schritt A erfasst, wobei in Schritt C das medizinische Analyseergebnis in Abhängigkeit dieser mehreren Zustandsinformationen bestimmt wird. Dies ermöglicht eine höhere Genauigkeit bei der Bestimmung des medizinischen Analyseergebnisses.

Der Begriff "Historische Gesundheitsinformation" bezeichnet im Sinne der vorliegenden Erfindung eine Gesamtheit von Informationen, die sich auf frühere oder dauerhaft gültige gesundheitsbezogene Merkmale, Zustände oder Ereignisse der zweiten menschlichen Person beziehen. Sie umfasst Daten, die vor einem aktuellen Erhebungszeitpunkt entstanden sind und die eine retrospektive oder kontinuierliche Beschreibung der gesundheitlichen Entwicklung, Konstitution oder Disposition einer Person ermöglichen. Mit anderen Worten bilden die historischen Gesundheitsinformationen die Grundlage eines virtuellen digitalen Zwillings (Digital Twin) einer echten menschlichen Person. Historische Gesundheitsinformationen können beispielsweise demografische Angaben (wie Alter und Geschlecht), genetische Merkmale (wie Genotypen oder Mutationen), anamnestische Daten (wie Vorerkrankungen, frühere Diagnosen, Therapien oder Operationen), biometrische Verlaufsdaten, Medikationshistorien, Impfstatus, familiäre Dispositionen, Lebensstilfaktoren sowie Ergebnisse früherer medizinischer Untersuchungen, Laboranalysen oder Bildgebungen umfassen. Die Informationen können aus strukturierten oder unstrukturierten Quellen stammen, durch Selbstangabe, medizinisches Fachpersonal oder technische Systeme erfasst worden sein.

Gemäß einer Ausführungsform des erfindungsgemäßen Arbeitsverfahrens werden in Schritt B mindestens zwei historische Gesundheitsinformationen erhalten, die sich bezüglich ihres Datentyps unterscheiden. Insbesondere werden in Schritt B sowohl Bilddaten als auch Textdaten erhalten und dann in Schritt C unter Verwendung der künstlichen Intelligenz gemeinsam analysiert. Zusätzlich können auch komplexe medizinische Daten und/oder Audiodaten und/oder Videodaten in Schritt B in Kombination erhalten werden und gemeinsam in Schritt C analysiert werden. Hierdurch kann ein umfassendes Bild eines Patienten erstellt und analysiert werden. Für die Analyse in Schritt C kann in diesem Fall insbesondere ein multimodales Model als künstliche Intelligenz oder als Teil der künstlichen Intelligenz verwendet werden.

Im Sinne dieser Erfindung können die historischen Gesundheitsinformationen so einen digitalen Zwilling der zweiten Person darstellen, sodass Therapieansätze durch Simulationen am digitalen Zwilling auf ihre Erfolgswahrscheinlichkeit hin getestet werden können. In Abhängigkeit von den Ergebnissen dieser Simulationen kann dann ein endgültiger Therapievorschlag als Nachricht der ersten menschlichen Person übermittelt werden.

Gemäß einer Ausführungsform des erfindungsgemäßen Arbeitsverfahrens umfasst das Verfahren folgenden weiteren Schritt E: Erfassen einer Feedback-Information der ersten menschlichen Person. Dabei wird die trainierte künstliche Intelligenz in Abhängigkeit von der Feedback-Information verändert, wobei die Veränderung der künstlichen Intelligenz vorzugsweise durch einen der folgenden Schritte erfolgt:
- Nachtrainieren der künstlichen Intelligenz, besipielsweise durch Feinabstimmung (z.B. engl. *supervised-, selfsupervised-, unsupervise fine-tuning*), oder Verstärkungslernen *(z.B. engl. reinforcement learning, reinforcement learning with human feedback*),
- Kontext-gestützte Individualisierung der künstlichen Intelligenz, sodass einem zukünftigen Prompt eine Kontextinformation, die auf der Feedback-Information basiert, hinzugegeben wird.

Kontext-gestützte Individualisierung der künstlichen Intelligenz bezeichnet im Sinne der vorliegenden Erfindung die Methode, eine trainierte künstliche Intelligenz - insbesondere große Sprachmodelle (LLMs), vortrainierte Foundation-Modelle oder multimodale KI-Systeme - gezielt an individuelle Anforderungen, Kontexte oder Benutzerbedürfnisse anzupassen, ohne das zugrunde liegende Modell selbst zu modifizieren. Stattdessen erfolgt die Steuerung des Verhaltens und der Antworten der künstlichen Intelligenz durch gezielte Eingaben (Prompts), die Kontext, Zielsetzung, Tonfall oder spezifisches Wissen enthalten. Eine hierzu zu zählende Methode ist Retrieval-Augmented Generation (RAG). Dabei wird der Prompt dynamisch durch relevante externe Informationen angereichert, die aus einer externen Wissensquelle (z. B. einer Dokumentensammlung, Datenbank oder einem individuellen Wissensspeicher) abgerufen werden. RAG erlaubt es der künstlichen Intelligenz, auf aktuelle, domänenspezifische oder benutzerspezifische Informationen zuzugreifen, die nicht im Trainingskorpus enthalten sind, und diese bei der Generierung ihrer Antwort zu berücksichtigen. Diese Form der Personalisierung ist besonders flexibel, da sie ohne aufwendiges Fine-Tuning auskommt und sich einfach an neue Kontexte oder Benutzer anpassen lässt.

"Nachtrainieren" bezeichnet den Prozess, bei dem ein bereits vortrainiertes künstliches Intelligenz-Modell durch zusätzliche Trainingsphasen optimiert wird, indem die Modellparameter angepasst werden. Dieser Prozess kann auf verschiedenen Methoden basieren: Beim überwachten Feinabstimmen (supervised fine-tuning) wird das Modell mit annotierten Daten weitertrainiert, um spezifische Aufgaben besser zu lösen. Beim unüberwachten Feinabstimmen (unsupervised fine-tuning) wird das Modell ohne explizite Labels auf nicht annotierten Daten trainiert, wobei es Muster und Strukturen eigenständig erkennt. Beim selbstüberwachten Feinabstimmen (self-supervised fine-tuning) lernt das Modell, indem es aus den Eingabedaten selbstständig Repräsentationen extrahiert, oft durch Verfahren wie Maskierung oder Rekonstruktion. Beim Verstärkungslernen ohne Feedback (reinforcement learning without feedback) wird das Modell durch Belohnung und Bestrafung aufgrund seiner eigenen Handlungen optimiert, ohne dass externes menschliches Feedback erforderlich ist. Verstärkendes Lernen mit menschlichem Feedback oder KI-Feedback (reinforcement learning with human or Al feedback) nutzt eine menschliche Interaktion bzw. ein KI-System, um dem Modell Rückmeldungen zu geben, die zu einer weiteren Verbesserung führen.

Im Gegensatz zu Kontext-gestützter Individualisierung, bei der keine Änderungen an den Modellparametern vorgenommen werden, führt das Nachtrainieren zu einer dauerhaften Veränderung der Modellgewichte und -strukturen.

Zur personalisierten Weiterentwicklung der künstlichen Intelligenz kann gemäß einem Ausführungsbeispiel ein Verfahren vorgesehen sein, bei dem menschliche Feedback-Informationen in das Verändern der künstlichen Intelligenz eingebunden wird. Die Feedback-Information dient hierbei der gezielten Anpassung des Modells an spezifische Anforderungen, Nutzungskontexte oder Qualitätskriterien. Gemäß einer Ausführungsform kann die Erfassung des Feedbacks entweder kontinuierlich während der Laufzeit des Systems oder in vordefinierten Zeitintervallen erfolgen, wobei die Art und Frequenz der Erhebung konfigurierbar ausgestaltet ist.

Um die Qualität und Vertrauenswürdigkeit des nachtrainierten künstlichen Intelligent sicherzustellen, kann eine Mechanik implementiert sein, welche eine Bewertung und ggf. Filterung der eingehenden Feedback-Informationen vornehmen. Diese Mechanik dient insbesondere dazu, verzerrtes, fehlerhaftes oder manipulatives Feedback zu identifizieren und vom Trainingsprozess auszuschließen. Die Bewertung kann automatisiert und/oder regelbasiert erfolgen. Zusätzlich kann die Bewertung derart ausgebildet sein, dass sie eine Einbeziehung zusätzlicher menschlicher Prüfungen erfordert. Auf diese Weise wird verhindert, dass unerwünschte Effekte wie Fehlinformationen, systematische Verzerrungen oder die Reproduktion unerwünschter Verhaltensmuster in der künstlichen Intelligenz verankert werden.

Optional kann das Verfahren eine Prüfschicht beinhalten, welche die Gültigkeit oder Plausibilität der Feedback-Information bewertet, bevor sie für die Veränderung der trainierten künstlichen Intelligenz herangezogen wird.

Gemäß einer Ausführungsform des erfindungsgemäßen Arbeitsverfahrens umfasst das Verfahren folgenden weiteren Schritt F: Regelmäßiges Erfassen von digitalen medizin-wissenschaftlichen Informationen aus einem digitalen Repository, insbesondere aus dem Internet, zum Beispiel über einen öffentlichen Pubmed-Server. Dabei wird die trainierte künstliche Intelligenz in Abhängigkeit von den medizin-wissenschaftlichen Informationen verändert, wobei die Veränderung der künstlichen Intelligenz vorzugsweise durch einen der folgenden Schritte erfolgt:
- Nachtrainieren der künstlichen Intelligenz, zum Beispiel durch eine Feinabstimmung (*engl. fine-tuning*) oder ein Verstärkungslernen *(engl. reinforcement learning*)*,*
- Kontext-gestützte Individualisierung der künstlichen Intelligenz, sodass einem zukünftigen Prompt eine Kontextinformation, die auf der Feedback-Information basiert, hinzugegeben wird.

Dieser Zugriff dient der Anreicherung der künstlichen Intelligenz mit domänenspezifischem Fachwissen, insbesondere im Hinblick auf medizinische Diagnose, Therapieempfehlungen, Wirkstoffforschung oder epidemiologische Zusammenhänge. Die Einbindung solcher externer Informationsquellen kann dynamisch oder dauerhaft erfolgen. Die trainierte künstliche Intelligenz wird durch den Zugriff befähigt, Inhalte aus den Quellen bei der Generierung von Analyseergebnissen zu berücksichtigen.

Zur Sicherstellung der inhaltlichen Validität der verwendeten medizinischen Informationen kann ein Qualitätssicherungsmechanismus - im Sinne eines Verfahrensschritts - vorgesehen sein, der eine Überprüfung der Quellenqualität umfasst. Dies kann durch eine formale Verifizierung der Herkunft, eine Bewertung auf Basis bibliometrischer Kennzahlen (z. B. Impact-Faktor, Zitationshäufigkeit), eine Kennzeichnung als Peer-Review-Publikation oder durch eine Kombination solcher Kriterien erfolgen. Der Vorteil dieses Mechanismus besteht darin, dass vorrangig wissenschaftlich fundierte und überprüfte Informationen genutzt werden. Damit wird die Zuverlässigkeit und Regeltreue der Ausgaben der künstlichen Intelligenz im medizinischen Kontext erhöht.

Darüber hinaus kann die künstliche Intelligenz so ausgestaltet sein, dass sie nicht nur bestehende Informationen verarbeitet, sondern selbstständig Muster, Korrelationen oder potenziell neue Zusammenhänge aus der Gesamtheit der eingebundenen wissenschaftlichen Publikationen ableiten kann. Diese Fähigkeit zur wissensgenerierenden Analyse stellt einen zusätzlichen funktionalen Vorteil dar. Die Mustererkennung kann regelbasiert, modellgestützt und/oder durch vergleichende Analyse realisiert werden.

Gemäß einer Ausführungsform des erfindungsgemäßen Arbeitsverfahrens wird die wahrnehmbare Nachricht in Schritt D in Echtzeit als Reaktion auf eine erfasste Umgebungsinformation erzeugt und der menschlichen Person wahrnehmbar zu Verfügung gestellt, die wahrnehmbare Nachricht kann gemäß einer Ausführungsform als Audionachricht über eine Lautsprechervorrichtung, insbesondere einen Kopfhörer oder ein Kopfhörerimplantat der menschlichen Person wahrnehmbar zu Verfügung gestellt werden.

Mit anderen Worten ist die trainierte künstliche Intelligenz derart ausgestaltet, dass sie Informationen (Umgebungsinformationen und/oder Zustandsinformationen und/oder historische Gesundheitsinformationen) in Echtzeit verarbeiten und darauf basierend Analyseergebnisse generieren kann. Dabei kann das Verfahren eine Methode zur Abschätzung und Einhaltung des Echtzeit-Zeitrahmens, in dem eine Nachricht gemäß Schritt D wahrnehmbar gemacht werden muss, umfassen. Die Bestimmung dieses Echtzeit-Zeitrahmens kann durch maschinelles Lernen, heuristische Verfahren oder vordefinierte statistische Modelle erfolgen, wobei der zulässige Antwortzeitraum anwendungs- oder nutzerspezifisch festlegbar sein kann.

Zur Erhöhung der Adaptivität kann vorgesehen sein, dass die trainierte künstliche Intelligenz in der Lage ist, den Echtzeit-Zeitrahmen dynamisch an veränderte Umgebungsbedingungen oder Nutzerinteraktionen anzupassen. Dies kann beispielsweise bei schwankender Netzwerklatenz, variierender Rechenleistung oder veränderlichem Nutzerverhalten (z. B. Reaktionszeiten, Unterbrechungen, Abbrüche) relevant werden. Die trainierte künstliche Intelligenz bewertet hierzu kontinuierlich systemrelevante Parameter und optimiert auf dieser Grundlage die zeitlichen Rahmenbedingungen ihrer Verarbeitungsvorgänge, um eine konsistente und kontextsensitive Echtzeitfähigkeit aufrechtzuerhalten.

Unter Echtzeit ist im Sinne der vorliegenden Anmeldung eine Zeitdifferenz zwischen dem Erfassen einer Umgebungsinformation oder einer Zustandsinformation einerseits und dem wahrnehmbaren Bereitstellen der Nachricht andererseits zu verstehen, die eine Beeinflussung zukünftiger Umgebungsinformationen oder Zustandsinformationen, die mit der vergangenen Umgebungsinformation oder Zustandsinformation im Zusammenhang stehen, ermöglichen. Ist die Umgebungsinformation beispielsweise eine digitale Tonaufnahme eines Anamnesegesprächs, so ist eine Zeitdifferenz von zum Beispiel 30 Sekunden zwischen dem Erfassen des bisherigen Anamnesegesprächs und dem Bereitstellen einer Nachricht, die beispielsweise auf Fragen aufmerksam macht, die der Arzt noch stellen sollte, als Echtzeit anzusehen. Da ein Anamnesegespräch in der Regel länger als 3 Minuten andauert, erlaubt das 30-sekündige Zeitfenster dem Arzt, das Anamnesegespräch in Abhängigkeit von der wahrnehmbaren Nachricht weiterzuführen.

Gemäß einer Ausführungsform des erfindungsgemäßen Arbeitsverfahrens umfasst das Verfahren folgenden weiteren Schritt G: Abschätzen eines Zeitraums, der durch einen Prozess, an dem die erste menschlichen Person beteiligt ist, beansprucht wird, beispielsweise einer Dauer eines Gesprächs, bei dem die erste menschliche Person beteiligt ist, in Abhängigkeit von der erfassten Umgebungsinformation. Dabei wird die wahrnehmbare Nachricht derart bereitgestellt wird, dass sich der Bereitstellungszeitpunkt innerhalb des in Schritt G) abgeschätzten Zeitraums befindet.

Gemäß einer Ausführungsform des erfindungsgemäßen Arbeitsverfahrens umfasst die künstliche Intelligenz ein Modell des maschinellen Lernens, vorzugsweise ein Sprachmodel, besonders bevorzugt ein multimodales Kl-Model inklusive Sprachmodell. Das Sprachmodell kann insbesondere ein großes Sprachmodell (Large Language Modell sein) sein. Die künstliche Intelligenz kann weiterhin insbesondere ein Foundation-Modell, insbesondere ein (sprachliches oder multimodales) Modell, das optional auf Transformer-Technologie oder einer anderen Modellarchitektur beruht, umfassen. Gemäß einer Ausführungsform können hierbei Large Language Modelle, hybride Expertenmodelle, Ensembling-Ansätze oder vergleichbare künstliche Intelligenz-Techniken zum Einsatz kommen.

Im Rahmen der vorliegenden Erfindung wird unter einem ,großen Sprachmodell' (engl. ,Large Language Model', LLM) ein künstliches Intelligenzsystem verstanden, das insbesondere ein künstliches neuronales Netzwerk mit einer Vielzahl von Parametern umfasst, typischerweise in einer Größenordnung von mehreren Milliarden Parametern. Das Modell ist auf der Grundlage umfangreicher Textkorpora oder sonstiger sprachlicher Daten trainiert, um Aufgaben der natürlichen Sprachverarbeitung (Natural Language Processing, NLP) auszuführen, wie etwa die Generierung, Analyse, Transformation oder Beantwortung natürlicher Spracheingaben.

Das große Sprachmodell kann insbesondere, aber nicht ausschließlich, auf autoregressiven, encoder-basierten, decoder-basierten oder encoder-decoder-basierten Architekturen beruhen, wie sie beispielsweise aus der sogenannten Transformer-Architektur bekannt sind. Der Begriff umfasst auch funktional äquivalente Systeme, die auf alternativen Architekturprinzipien beruhen, sofern sie eine vergleichbare Fähigkeit zur kontextabhängigen Verarbeitung und Generierung natürlicher Sprache aufweisen.

Das große Sprachmodell kann ferner in der Lage sein, Eingaben in gesprochener Sprache, Textform oder strukturierten Formaten entgegenzunehmen und in geeigneter Weise zu verarbeiten. Die Nutzung solcher Sprachmodelle kann in vielfältiger Weise erfolgen, z. B. als Bestandteil eines Assistenzsystems, eines Dialogsystems, einer Textklassifikation, einer maschinellen Übersetzung oder eines allgemeinen Sprachverständnissystems.

Ein "multimodales Modell" im Sinne der vorliegenden Erfindung bezeichnet ein computer-implementiertes System, das derart ausgebildet ist, Eingangsdaten aus mehreren unterschiedlichen Datenmodalitäten - beispielsweise Text, Bild, Sprache, numerische Werte, zeitabhängige Signale, strukturierte Tabellen oder medizinische komplexe Daten - gemeinsam zu verarbeiten, zu analysieren oder zu interpretieren. Das Modell kann auf neuronalen Netzwerken basieren, die domänenspezifische Merkmalsrepräsentationen aus verschiedenen Modalitäten extrahieren und in einer gemeinsamen latenten Repräsentation integrieren. Diese Integration ermöglicht es, kontextübergreifende Zusammenhänge zu erfassen, komplexe semantische Beziehungen zwischen Modalitäten herzustellen und auf dieser Grundlage Klassifikationen, Vorhersagen, Generierungen oder Entscheidungsprozesse vorzunehmen.

Gemäß einer Ausführungsform des erfindungsgemäßen Arbeitsverfahrens sind bei der Durchführung des Verfahrens die erste menschliche Person und die zweite menschliche Person ein und dieselbe menschliche Person. In einer weiteren Ausführungsform kann vorgesehen sein, dass die Anpassung der durch die künstliche Intelligenz generierten Nachricht in Schritt D an die jeweilige Zielperson unter Einbeziehung individueller Daten erfolgt. Beispielsweise können auch mehrsprachige oder vereinfachte Sprachversionen generiert werden, falls die Zielperson dies bevorzugt oder das Bedürfnis hat. Die Individualisierung der Nachricht kann dabei auf Grundlage zuvor erfasster Daten erfolgen, welche personenbezogene Muster, Präferenzen oder Verhaltensweisen abbilden, oder unter Verwendung von in Echtzeit erfassten Sensordaten (zum Beispiel Daten mit Umgebungsinformationen oder Zustandsinformationen), welche den aktuellen Zustand oder Kontext der Person widerspiegeln. Durch die kombinierte oder alternative Nutzung dieser Datenquellen kann eine optimierte, kontextsensitive und personalisierte Ansprache erzielt werden, welche insbesondere im Hinblick auf Wirksamkeit, Relevanz und Akzeptanz der übermittelten Nachricht verbessert ist.

Gemäß einer Ausführungsform des erfindungsgemäßen Arbeitsverfahrens wird in Schritt C) zumindest ein medizinischer Verhaltensvorschlag bestimmt, wobei die wahrnehmbare Nachricht derart ausgebildet ist, dass die wahrnehmbare Nachricht den medizinischen Verhaltensvorschlag für die menschliche Person erfassbar macht und
- die wahrnehmbare Nachricht für die menschlichen Person zeitlich derart erfassbar gemacht wird, dass ein erstes Verhaltensmuster der menschlichen Person durch die Nachricht beeinflussbar ist; oder
- die wahrnehmbare Nachricht für die menschlichen Person zeitlich erfassbar gemacht wird, um einen messbaren Zustandsparameter der menschlichen Person, wie zum Beispiel die Herzfrequenz, zu regeln.

Die Art der Beeinflussung kann dabei sowohl in Form direkter Anweisungen als auch durch subtile Interventionen erfolgen, beispielsweise durch gezielte Modulation von Hintergrundgeräuschen, Änderung visueller Reize oder Variation sensorischer Umgebungsparameter. Dies ermöglicht eine differenzierte, situationssensible Steuerung des Nutzerverhaltens unter Berücksichtigung kognitiver und emotionaler Verarbeitungsmechanismen.

Zur weiteren Optimierung der Verhaltensvorschläge kann eine Anpassungsmechanik vorgesehen sein, welche adaptives Lernen auf Basis von Rückmeldungen oder wiederkehrenden Nutzungsmustern ermöglicht. Hierzu kann die trainierte künstliche Intelligenz insbesondere Nutzerreaktionen, explizite Rückmeldungen (z. B. Bewertungen) oder implizite Parameter wie langfristig beobachtbare physiologische Veränderungen (z. B. Stressindikatoren, Entspannungsniveaus) analysieren, um die Effektivität künftiger Interventionen zu steigern. Dadurch wird eine kontinuierliche, personalisierte Weiterentwicklung der Verhaltensvorschläge im Sinne eines lernenden Systems erreicht.

Gemäß einer Ausführungsform des erfindungsgemäßen Arbeitsverfahrens sind die erste menschliche Person und die zweite menschliche Person unterschiedliche Personen. Die Struktur der Beziehung von erster menschlicher Person und zweiter menschlicher Person kann insbesondere medizinischer, therapeutischer oder beratender Natur sein, wie etwa im Rahmen eines Arzt-Patient-, Therapeut-Klient- oder Coach-Coachee-Verhältnisses. Die Nachricht gemäß Schritt D an die erste menschliche Person (Arzt, Therapeut, Coach) kann dabei Informationen, Empfehlungen oder Hinweise enthalten, die sich auf den Zustand oder das Verhalten der zweiten menschlichen Person (Patient, Klient, Coachee) beziehen und zur Unterstützung der betreuenden oder beratenden Tätigkeit des Empfängers dienen.

Zur Wahrung des Datenschutzes und zur Sicherstellung der Datensicherheit bei der Kommunikation solcher personenbezogenen Inhalte zwischen den beteiligten Akteuren kann eine zusätzliche Absicherungseinrichtung vorgesehen sein. Diese kann insbesondere durch den Einsatz verschlüsselter Kommunikationsprotokolle, Zugangskontrollen oder durch dezentrale Speicherung sensibler Daten realisiert werden. Dies hat den Vorteil, dass die unbefugte Einsichtnahme, Manipulation oder Verfälschung personenbezogener Informationen wirksam verhindert und gleichzeitig die Integrität und Vertraulichkeit der übermittelten Inhalte gewährt werden kann.

Gemäß einer Ausführungsform des erfindungsgemäßen Arbeitsverfahrens ist die mindestens eine digitale historische Gesundheitsinformation der zweiten menschlichen Person ausgewählt aus einer der folgenden digitalen Informationen:
- Textdaten oder Bilddaten von schriftlichen medizinischen Befunden, insbesondere Arztbriefen oder Patientenbriefen;
- Strukturelle Daten oder Bilddaten von Blutwerten;
- Strukturelle Daten oder Bilddaten zum Impfstatus;
- Strukturelle Daten oder Bilddaten von Medikationsplänen;
- Bilddaten von bildgebenden medizinischen Untersuchungsverfahren;
- Genetische Daten (Gensequenzen);
- Daten über das Mikrobiom.

Gemäß einer Ausführungsform des erfindungsgemäßen Arbeitsverfahrens umfasst die mindestens eine digitale historische Gesundheitsinformation der zweiten menschlichen Person medizinische komplexe Daten. "Komplexe Daten" im Sinne der vorliegenden Erfindung bezeichnen medizinisch-biologische Datensätze, die sich durch eine hohe strukturelle, funktionale und/oder semantische Komplexität auszeichnen. Dazu zählen insbesondere - jedoch nicht ausschließlich - genomische, transkriptomische, proteomische, epigenetische, metagenomische, mikrobiologische sowie weitere sogenannte Omics-Datensätze. Diese können Sequenzinformationen (z. B. DNA, RNA, Protein), Expressionsprofile, epigenetische Modifikationen (z. B. Methylierungsmuster, Histonmodifikationen), Daten zur Proteinstruktur und -interaktion, Zusammensetzungen mikrobieller Gemeinschaften (z. B. Mikrobiom-Daten) sowie deren Verknüpfungen mit klinischen, phänotypischen oder bildgebenden Informationen umfassen.

Gemäß einer Ausführungsform des erfindungsgemäßen Arbeitsverfahrens werden in Schritt B mindestens zwei unterschiedliche Gesundheitsinformationen, vorzugsweise mindestens drei unterschiedliche Gesundheitsinformationen erhalten, wobei in Schritt C das Bestimmen des digitalen medizinischen Analyseergebnisses in Abhängigkeit von den mindestens zwei in Schritt B erhaltenen Gesundheitsinformationen, vorzugsweise in Abhängigkeit von den mindestens drei in Schritt B erhaltenen Gesundheitsinformationen, erfolgt. Dabei kann eine Priorisierung vorgesehen sein, sodass eine erste Gesundheitsinformation bei dem Bestimmen des medizinischen Analyseergebnisses stärker gewichtet wird als eine zweite Gesundheitsinformation.

In einer weiteren Ausführungsform kann ein Mechanismus vorgesehen sein, der die Konsistenz und Plausibilität der verarbeiteten Gesundheitsinformationen überprüft. Dieser Mechanismus dient dazu, fehlerhafte, unplausible oder widersprüchliche Gesundheitsinformationen zu identifizieren und gegebenenfalls aus dem weiteren Verarbeitungsprozess auszuschließen oder entsprechend zu kennzeichnen. Die Überprüfung kann regelbasiert, modellgestützt oder unter Verwendung statistischer Methoden erfolgen und trägt zur Erhöhung der Zuverlässigkeit, Genauigkeit und Sicherheit der datengestützten Entscheidungen und Ausgaben der künstlichen Intelligenz bei.

Darüber hinaus kann die Verarbeitung der Gesundheitsinformationen so ausgestaltet sein, dass die Anzahl und Auswahl der einbezogenen Gesundheitsinformationen dynamisch anpassbar ist. Dies ermöglicht eine skalierbare Nutzung von Gesundheitsinformationen, abhängig von deren aktueller Verfügbarkeit, Qualität und diagnostischer Relevanz. Auf diese Weise kann das System sowohl bei umfassend dokumentierten als auch bei eingeschränkt verfügbaren Gesundheitsinformationen sinnvoll eingesetzt werden, ohne dass die Funktionsfähigkeit oder Aussagekraft wesentlich beeinträchtigt wird.

Gemäß einer Ausführungsform des erfindungsgemäßen Arbeitsverfahrens ist die erste Computerdatenbank innerhalb einer ersten Serverumgebung angeordnet, wobei die künstliche Intelligenz innerhalb einer zweiten, sich von der ersten Serverumgebung unterscheidenden Serverumgebung angeordnet ist, sodass die künstliche Intelligenz derart abgeschirmt ist, dass die künstliche Intelligenz nicht unmittelbar und/oder nicht beständig auf die in der ersten Computerdatenbank abgelegten historischen Gesundheitsinformationen zugreifen kann, wobei die künstliche Intelligenz vorzugsweise derart abgeschirmt ist, dass die künstliche Intelligenz nicht auf das Internet zugreifen kann.

Der Datenaustausch zwischen der ersten Serverumgebung und der zweiten Serverumgebung kann über definierte Schnittstellen, insbesondere API-Schnittstellen, erfolgen, die mit rollenbasierten oder inhaltlich beschränkten Zugriffskontrollen ausgestattet sind. Dadurch wird sichergestellt, dass ausschließlich autorisierte Systeme auf relevante Gesundheitsinformationen zugreifen und diese verarbeiten können.

Zur weiteren Erhöhung der Datensicherheit und zur Sicherstellung der Integrität der verarbeiteten Gesundheitsinformationen kann zudem eine dezentrale Speicherung vorgesehen sein. Hierbei können Technologien wie die Blockchain oder vergleichbare verteilte Datenhaltungssysteme eingesetzt werden, um eine nachträgliche Manipulation zu verhindern und die Nachvollziehbarkeit von Zugriffen oder Änderungen zu gewährleisten. Dieses Sicherheitskonzept ergänzt bestehende Zugriffsbeschränkungen und bietet ein erhöhtes Maß an technischer und organisatorischer Absicherung im Umgang mit sensiblen Gesundheitsinformationen.

Insgesamt erhöht dies die Datensicherheit und schützt die in der ersten Serverumgebung abgelegten Gesundheitsdaten vor unbefugtem Zugriff. Die Trennung stellt zudem sicher, dass das trainierte Modell in der zweiten Serverumgebung nur temporär auf die Daten zugreifen kann und damit das Risiko einer dauerhaften Speicherung sensibler Daten minimiert wird.

Gemäß einer Ausführungsform des erfindungsgemäßen Arbeitsverfahrens werden Gesundheitsinformationen, die zum Zwecke der Verarbeitung durch die künstliche Intelligenz in die zweite Serverumgebung übertragen werden, ausschließlich temporär in der zweiten Serverumgebung gespeichert werden, um von der künstlichen Intelligenz als Eingangsgrößen verwendet zu werden, wobei die Gesundheitsinformationen vorzugsweise ausschließlich quasi-anonymisiert in der zweiten Serverumgebung gespeichert werden, um von der künstlichen Intelligenz als Eingangsgrößen verwendet zu werden.

Dies ermöglicht, die Gesundheitsinformationen lediglich für die Dauer der jeweiligen Verarbeitungsvorgänge vorzuhalten. Nach Abschluss der Verarbeitung oder nach dem Erreichen eines vordefinierten Zeitraums erfolgt eine automatisierte Löschung der temporär gespeicherten Daten. Dieser Ansatz unterstützt die Einhaltung datenschutzrechtlicher Vorgaben und minimiert das Risiko eines unbefugten Zugriffs auf gespeicherte Gesundheitsinformationen.

In einer weiteren Ausführungsform kann die temporäre Speicherung von Gesundheitsinformationen innerhalb der zweiten Serverumgebung um zusätzliche Sicherheitsschritte ergänzt werden, um auch in Ausnahmesituationen wie einem unerwarteten Systemabsturz, Stromausfall oder einem potenziellen Datenleck die Löschung sensibler Inhalte sicherzustellen. Hierzu kann ein Sicherheitsmechanismus vorgesehen sein, der im Falle eines Systemfehlers automatisiert eingreift und gespeicherte Gesundheitsinformationen zuverlässig entfernt oder unlesbar macht, um eine über die vorgesehene Speicherdauer hinausgehende Verfügbarkeit auszuschließen.

Darüber hinaus kann vorgesehen sein, dass bestimmte Gesundheitsinformationen, die temporär verarbeitet wurden, pseudonymisiert oder anonymisiert für nachgelagerte Zwecke verwendet werden, insbesondere zur Qualitätssicherung des Verfahrens bzw. Systems oder zur modellbasierten Weiterentwicklung der künstlichen Intelligenz. Eine solche Nutzung erfolgt ausschließlich unter der Voraussetzung, dass ein Personenbezug dauerhaft ausgeschlossen ist und geltende Datenschutzrichtlinien eingehalten werden. Auf diese Weise kann eine Balance zwischen funktionaler Systemoptimierung und dem Schutz personenbezogener Gesundheitsinformationen gewährleistet werden.

Gemäß einer Ausführungsform des erfindungsgemäßen Arbeitsverfahrens wird in Schritt C ein Therapievorschlag bestimmt. Das Verfahren umfasst dabei folgenden weiteren Schritt: Simulieren einer körperlichen Reaktion der zweiten menschlichen Person auf den in Schritt C bestimmten Therapievorschlag in Abhängigkeit von zumindest einer erhaltenen Gesundheitsinformation der zweiten menschlichen Person, sodass ein Simulationsergebnis eine Erfolgswahrscheinlichkeit des Therapievorschlags umfasst, wobei der in Schritt C bestimmte Therapievorschlag in Abhängigkeit von dem Simulationsergebnis verändert oder beibehalten wird, bevor die Nachricht in Schritt D in Abhängigkeit von dem Therapievorschlag erzeugt.

Das Verfahren kann zudem den Schritt des Simulierens einer körperlichen Reaktion oder eines Gesundheitsverlaufs der zweiten menschlichen Person mithilfe eines digitalen Zwillings (Digital Twin), der auf den historischen Gesundheitsinformationen basiert, wobei dieser digitale Zwilling als prädikatives Modell fungieren kann, das als Approximation physiologischer und/oder psychologischer Prozesse der zweiten Person dient und auf künstlicher Intelligenz basieren kann, wobei die für den digitalen Zwilling herangezogenen Modellparameter in Echtzeit oder intervallweise durch real erfasste Zustandsinformationen aktualisiert werden können, was Teil eines Echtzeit-Feedback-Abgleichs sein kann, bei dem Simulationsergebnisse mit neu eingehenden Realdaten der zweiten Person verglichen werden, um das dem digitalen Zwilling zugeordnete Modell oder einen zugeordneten individuenspezifischen Datenspeicher kontinuierlich zu verfeinern und zu trainieren, wodurch die Fähigkeit zur Verbesserung präziser Vorhersagen verbessert wird. Die Simulation erfolgt in Abhängigkeit von zumindest einer erhaltenen Gesundheitsinformation der zweiten menschlichen Person und liefert ein Simulationsergebnis, das eine Erfolgswahrscheinlichkeit des Therapievorschlags umfasst. Ziel dieser Simulation ist es, die potenziellen Wirkungen des Therapievorschlags am digitalen Zwilling - als eine auf erlernten Parametern basierende Abstraktion der Prozesse der zweiten Person - zu simulieren, bevor dieser in der Realität umgesetzt wird. In Abhängigkeit dieses Simulationsergebnisses wird der in Schritt C bestimmte Therapievorschlag wahlweise verändert oder beibehalten, bevor die Nachricht in Schritt D in Abhängigkeit von dem Therapievorschlag erzeugt wird. Auch dieser Prozess dient primär der Bereitstellung prädikativer Informationen zur Bewertung des Vorschlags und kann bei der Entscheidungsfindung, zur Reduzierung von Unsicherheiten, Erkennen von notwendigen Interventionsmaßnahmen oder -zeitpunkten oder zur Bewertung von Abweichungen dienen.

Die der Erfindung zugrundeliegende Aufgabe wird auch gelöst durch ein computer-implementiertes Verfahren zum Spezifizieren einer künstlichen Intelligenz, wobei die trainierte künstliche Intelligenz mindestens ein trainiertes oder teiltrainiertes Foundation-Modell (z.B. ein Large Language Model oder ein multimodales Modell) umfasst, welches Text, Bild, Audio, genetische Sequenzen oder andere Datenformate verarbeiten kann. In einem ersten Spezifizierungsschritt wird der trainierten künstlichen Intelligenz mithilfe der Methode RAG (Retrieval-Augmented Generation) spezifisches Wissen angeeignet wird, wobei gemäß einer Ausführungsform das Foundation-Modell im ersten Spezifizierungsschritt Textinformationen aus einer ersten Datenquelle und Textinformationen aus einer zweiten Datenquelle erhält, wobei gemäß einer Ausführungsform das Foundation-Modell im ersten Spezifizierungsschritt zusätzlich eine Priorisierungsinformation bezüglich der ersten Datenquelle und der zweiten Datenquelle erhält, sodass das Foundation-Modell Textinformationen aus der ersten Datenquelle stärker gewichtet als Textinformationen aus der zweiten Datenquelle, wobei die erste Datenquelle und die zweite Datenquelle derart ausgewählt sind, dass sie medizinische, insbesondere humanmedizinische Fachinformationen, oder weitere domänespezifische Daten (z.B. Proteinstrukturen, molekulare Sequenzen) enthalten können, wobei die erste Datenquelle vorzugsweise medizinische Leitlinien von medizinischen Fachgesellschaften umfasst.

RAG (Retrieval-Augmented Generation) ermöglicht es, extern gespeicherte Informationen in Echtzeit dynamisch abzurufen und in den Generierungsprozess einzubinden, wodurch die trainierte künstliche Intelligenz, welche gemäß einer Ausführungsform ein großes Sprachmodell (LLM) sein kann, stets auf aktuelle Fachinformationen zugreifen kann.

Die der Erfindung zugrundeliegende Aufgabe wird auch gelöst durch ein System zum Beeinflussen eines Verhaltens einer ersten menschlichen Person, wobei das System eine Datenverarbeitungseinheit umfasst, wobei die Datenverarbeitungseinheit derart ausgebildet und eingerichtet ist, dass sie die oben genannten Schritte A, B und C durchführt, wobei das System eine Übermittlungseinheit umfasst, wobei die Übermittlungseinheit derart ausgebildet und eingerichtet ist, dass sie den oben genannten Schritt D durchführt. Das System kann derart ausgebildet sein, dass es eines oder mehrere der zuvor beschriebenen Verfahren ausführt. Merkmale, die nur in Bezug auf das erfindungsgemäße Verfahren beschrieben worden sind, sind auch Merkmale eines hierfür entsprechend ausgebildeten Systems.

Die Übermittlungseinheit kann dabei unterschiedliche Ausgestaltungen aufweisen, beispielsweise in Form drahtloser Kommunikationsmodule, mobiler Endgeräte, haptischer Feedbackgeräte, akustischer Ausgabesysteme oder visueller Darstellungsmedien wie Augmented-Reality-Brillen oder Bildschirmen.

Zur Erweiterung der Systemfunktionalität kann ferner eine sichere Schnittstelle zur Interaktion mit externen Informationssystemen vorgesehen sein. Hierzu zählen insbesondere Krankenhausinformationssysteme, elektronische Patientenakten, Telemedizin-Plattformen oder vergleichbare medizinische Dateninfrastrukturen. Die Schnittstelle kann unter Einsatz gängiger Standards sowie unter Anwendung verschlüsselter Datenübertragungsverfahren ausgestaltet sein, um eine nahtlose, datenschutzkonforme Integration in bestehende IT-Umgebungen zu ermöglichen. Dadurch wird die Interoperabilität des Systems erhöht und eine konsistente, kontextbezogene Informationsverarbeitung im medizinischen Ökosystem sichergestellt.

Die der Erfindung zugrundeliegende Aufgabe wird auch gelöst durch ein Computerprogammprodukt, wobei das Computerprogrammprodukt Befehle umfasst, die bei der Ausführung des Programms durch einen Computer diesen veranlassen, eines der zuvor beschriebenen Verfahren, insbesondere die Schritte A, B, C und D auszuführen.

Die der Erfindung zugrundeliegende Aufgabe wird auch gelöst durch ein computerlesbares Speichermedium, umfassend Befehle, die bei der Ausführung durch einen Computer diesen veranlassen, eines der zuvor beschriebenen Verfahren, insbesondere die Schritte A, B, C und D auszuführen.

Weitere Merkmale, Beispiele und Vorteile der vorliegenden Erfindung werden ersichtlich anhand der Figuren und der dazugehörigen nachfolgenden Beschreibung. Es zeigen:
- Figur 1:: eine erste Ausführungsform des erfindungsgemäßen computer-implementierten Verfahrens, bei dem die erste menschliche Person und die zweite menschliche Person unterschiedliche Personen sind,
- Figur 2:: eine zweite Ausführungsform des erfindungsgemäßen Verfahrens, bei dem die erste menschliche Person und die zweite menschliche Person ein- und dieselbe Person ist.

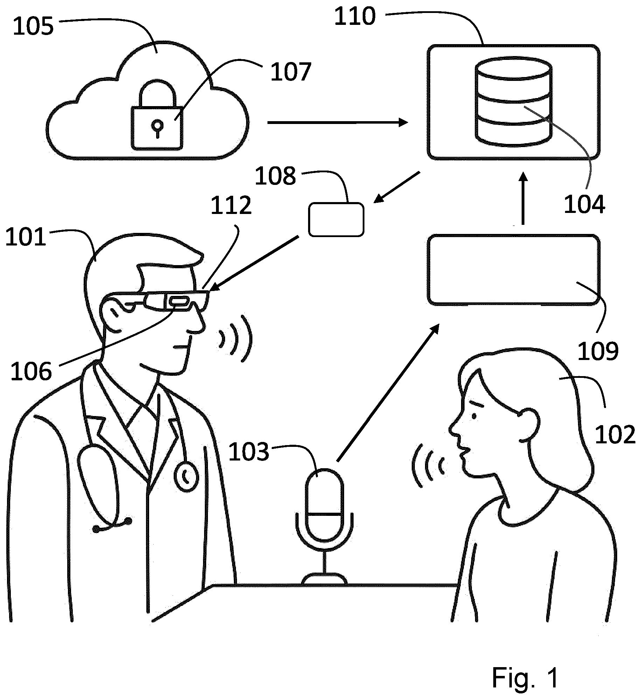

Figur 1 zeigt eine schematische Darstellung eines beispielhaften Ausführungsbeispiels des erfindungsgemäßen Verfahrens und Systems im Anwendungskontext einer medizinischen Beratungssituation, zum Beispiel eines Anamnese-Gespräch.

In der Darstellung ist eine erste Person 101, beispielsweise ein Arzt oder eine medizinische Fachkraft, einer zweiten Person 102, beispielsweise einer Patientin, zugewandt. Die beiden Personen führen ein Gespräch, wobei ein Mikrofon 103 in der Umgebung oder am Körper der ersten Person 101 positioniert ist und das Gespräch aufzeichnet. Die aufgenommenen Audiodaten 109 sind Umgebungsinformationen im Sinne der vorliegenden Erfindung und werden als Signal an eine erste Datenverarbeitungseinheit 104 übertragen (Schritt A).

Diese Datenverarbeitungseinheit 104 befindet sich in der zweiten Serverumgebung 110 und beinhaltet die trainierte künstliche Intelligenz, hier im Speziellen ein Large Language Model.

Zusätzlich existiert die erste Serverumgebung 105 (hier als gesicherter Cloud-Speicher), welche die erste Computerdatenbank mit den historischen Gesundheitsinformationen 107 der zweiten Person 102 (hier: der Patientin) beinhaltet. Die erste Serverumgebung 105 steht in Verbindung mit der zweiten Serverumgebung 110 und der darin enthaltenen Datenverarbeitungseinheit 104, sodass dort sowohl die Audiodaten 109 als auch die historischen Gesundheitsinformationen 107 zusammengeführt (Schritt B) und verarbeitet werden.

Somit werden sowohl die Umgebungsinformationen, in Form von Audiodaten 109, als auch die historischen Gesundheitsinformationen 107 der Patientin, zum Beispiel ein vom Hausarzt hinterlegter Medikamentenplan, der zweiten Serverumgebung 110 und dem darin enthaltenen Large Language Model zugänglich gemacht. Das Large Language Model kann dabei dahingehend spezifiziert worden sein, beispielweise mittels RAG, dass es auf die Leitlinien zur Medikation von Krankheiten, die im mit dem Mikrofon 103 erfassten Gespräch angesprochen werden, Zugriff hat und diese Leitlinien für die Ausgabe von Empfehlungen priorisiert. Die trainierte künstliche Intelligenz analysiert dann fortlaufend den Verlauf des medizinischen Beratungsgesprächs vor dem Hintergrund dieser Leitlinien und des bekannten Medikamentenplans. Kommt es dann beispielweise zu einer Inkonsistenz, listet beispielweise die Patientin Medikamente auf, die sie regelmäßig einnimmt, die aber nicht im Medikamentenplan hinterlegt sind, bestimmt die trainierte künstliche Intelligenz ein medizinisches Analyseergebnis 108, welches diese Inkonsistenz zum Inhalt hat. Dieses Analyseergebnis 108 wird dann dem hier gezeigten medizinischen Fachpersonal, also der ersten Person 101, in Echtzeit übermittelt, sodass für das Fachpersonal die Möglichkeit besteht, die Patientin 102 noch während des Beratungsgesprächs auf die Inkonsistenz hinzuweisen. Dies kann zu einer schnellen und effizienten Aufklärung offener medizinischer Fragen führen, die ansonsten oftmals nur nachträglich und teils mit schweren gesundheitlichen Folgen beantwortet werden.

Die Übermittlungseinheit für die ersten Person 101 ist im hier gezeigten Beispiel eine Brille 112 mit integriertem Display 106, welche die im Schritt D erzeugte Nachricht, insbesondere eine Rückmeldung oder Empfehlung, auf Basis des im Schritt C bestimmten medizinischen Analyseergebnisses 108 anzeigt. Das medizinische Analyseergebnis 108 wird von der künstlichen Intelligenz innerhalb der zweiten Serverumgebung 110 in Abhängigkeit von den erhaltenen Umgebungsinformationen (Audiodaten 109) und den historischen Gesundheitsinformationen 107 der Patientin 102 ermittelt. Die Anzeigen der Nachricht gegenüber der ersten Person 101 und somit das Kommunizieren des Analyseergebnisses 108 der verarbeiteten Umgebungs- und Gesundheitsinformationen erfolgt visuell über die Brille 112.

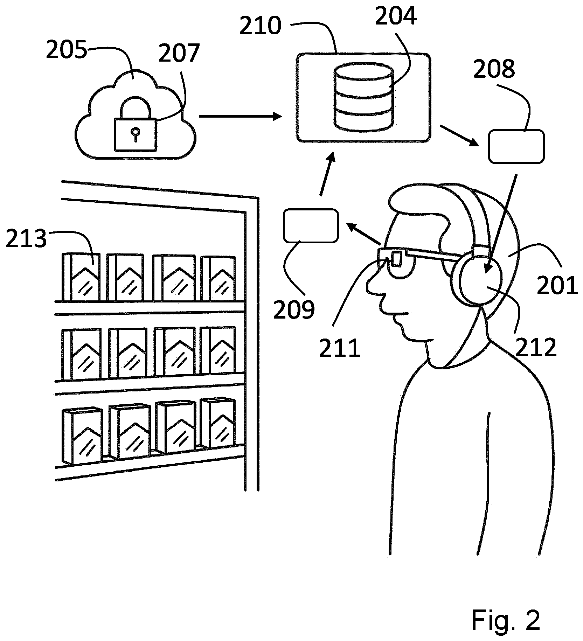

Figur 2 zeigt eine schematische Darstellung eines beispielhaften Ausführungsbeispiels eines erfindungsgemäßen Verfahrens und Systems zur situationsbezogenen Informationsverarbeitung und Nachrichtenausgabe im Kontext einer gesundheitsbezogenen Verhaltensintervention.

Hier ist eine Einzelperson 201 erkennbar, die im Sinne der vorliegenden Erfindung sowohl die erste menschliche Person als auch die zweite menschliche Person ist und die sich im gezeigten Kontext vor einem Regal mit Zigarettenpackungen 213 befindet. Die Person trägt eine Augmented-Reality-Brille 211 mit integrierter Kameraeinheit, über welche die visuelle Umgebung erfasst wird. Die dabei erzeugten Bild- oder Videodaten 209 werden als Umgebungsinformationen an die in der zweiten Serverumgebung 210 angeordnete Datenverarbeitungseinheit 204 übertragen (Schritt A).

Parallel dazu ist die erste Serverumgebung 205 mit der ersten Computerdatenbank vorgesehen. Auf der ersten Computerdatenbank sind historische Gesundheitsinformationen 207 der Person 201 abgelegt, beispielweise Ergebnisse eines Lungenfunktionstest oder AnamneseGesprächs-Protokolle, aus denen eine Raucherhistorie (zum Beispiel der auf die Person bezogene Wert "packyears") hervorgeht.

Die erste Serverumgebung 205 steht mit der zweiten Serverumgebung 210 und der darin enthaltenen Datenverarbeitungseinheit 204 in Verbindung, sodass die Datenverarbeitungseinheit zumindest temporär auf diese Gesundheitsinformationen zugreifen kann (Schritt B).

Das hier beschriebene Verfahren kann im gezeigten Kontext dann konkret wie folgt ablaufen: Über die Videodaten 209 erkennt die in der zweiten Serverumgebung 210 ansässige trainierte künstliche Intelligenz, dass sich die Person 201 einem Regal mit Zigarettenpackungen 213 nähert. Ggf. erfährt die trainierte künstliche Intelligenz auch über Zustandsinformationen wie einem am Handgelenk gemessen Pulsschlag der Person 201 (nicht gezeigt), dass sich das Erregungslevel der Peron 201 erhöht. Gleichzeitig erhält die trainierte künstliche Intelligenz von der ersten Computerdatenbank die historische Gesundheitsinformation 207, dass sich das Raucherverhalten der Person in den letzten Monaten deutlich gebessert/reduziert hat. Die trainierte künstliche Intelligenz führt darauf eine erste Simulation des Gesundheitsverlaufs der Person unter der Annahme, dass die Person 201 jetzt eine Zigarettenpackung 213 kauft und diese zeitnah konsumiert, durch. Davor, danach oder parallel hierzu wird eine zweite Simulation unter der Annahme, dass die Person keine Zigaretten konsumiert, durchgeführt. Das medizinische Analyseergebnis 208 ist in diesem Fall das Vergleichsergebnis dieser beiden Simulationen. Die trainierte künstliche Intelligenz ist dann im gezeigten Beispiel derart voreingestellt, dass sie weiß, dass die Person 201 gerne zahlenbasierte Entscheidungen trifft. Daher erstellt sie eine Nachricht, in der die Auswirkungen eines Zigarettenkonsums auf spezifische Gesundheitsparameter der Person wie das Fitnesslevel oder die Lungenfunktion mitgeteilt werden und wie sich diese Paramater ohne einen entsprechenden Konsum verhalten würden. Diese Nachricht wird der Person 201 im gezeigten Beispiel über die Kopfhörer 212 vorgespielt, um so schließlich eine Gesundheitsprävention im entscheidenden Moment zu ermöglichen.

Für Zwecke der ursprünglichen Offenbarung wird darauf hingewiesen, dass sämtliche Merkmale, bzw. Ausführungsformen wie sie sich aus der vorliegenden Beschreibung, den Zeichnungen und den Ansprüchen für einen Fachmann erschließen, auch wenn sie konkret nur im Zusammenhang mit bestimmten weiteren Merkmalen beschrieben wurden, sowohl einzeln als auch in beliebigen Zusammenstellungen mit anderen der hier offenbarten Merkmale oder Merkmalsgruppen kombinierbar sind, soweit dies nicht ausdrücklich ausgeschlossen wurde oder technische Gegebenheiten derartige Kombinationen unmöglich oder sinnlos machen. Auf die umfassende, explizite Darstellung sämtlicher denkbarer Merkmalskombinationen wird hier nur der Kürze und der Lesbarkeit der Beschreibung wegen verzichtet.

Während die Erfindung im Detail in den Zeichnungen und der vorangehenden Beschreibung dargestellt und beschrieben wurde, erfolgt diese Darstellung und Beschreibung lediglich beispielhaft und ist nicht als Beschränkung des Schutzbereichs gedacht, so wie er durch die Ansprüche definiert wird. Die Erfindung ist nicht auf die offenbarten Ausführungsformen beschränkt.

## Patentansprüche

1. Computer-implementiertes Arbeitsverfahren zum Erzeugen einer mit menschlichen Sinnesorgangen wahrnehmbaren Nachricht für eine erste menschliche Person, wobei das Verfahren folgende Schritte umfasst:
A) Dauerhaftes oder wiederholtes Erhalten von mindestens einer zeitlich veränderlichen und mit einem Mobilgerät messbaren digitalen Umgebungsinformation der ersten menschlichen Person und/oder der zweiten menschlichen Person, wie zum Beispiel geobasierten Informationen (GPS-Signale, WLAN -Standorte) oder einer digitalen akustischen Information in Form einer digitalen Sprach- und/oder Tonaufnahme eines Mikrofons,
und/oder
dauerhaftes oder wiederholtes Erhalten von mindestens einer digitalen zeitlich veränderlichen Zustandsinformation einer zweiten menschlichen Person wie zum Beispiel der gemessenen Herzfrequenz der zweiten menschlichen Person;
B) Erhalten von mindestens einer digitalen historischen Gesundheitsinformation der zweiten menschlichen Person, wie zum Beispiel dem Impfstatus der zweiten menschlichen Person oder dem aktuellen Medikationsplan der zweiten menschlichen Person, aus einer ersten Computerdatenbank;
C) Bestimmen eines digitalen medizinischen Analyseergebnisses, insbesondere eines Anamneseergebnisses der zweiten menschlichen Person und/oder einer medizinischen Diagnose der zweiten menschlichen Person und/oder eines medizinischen Verhaltensvorschlags für die zweiten menschliche Person und/oder eines medizinischen Therapievorschlag für die zweite menschliche Person,
• in Abhängigkeit von der in Schritt A erhaltenen Umgebungsinformation, falls in Schritt A eine Umgebungsinformation erhalten worden ist, und/oder in Abhängigkeit von der in Schritt A erhaltenen Zustandsinformation, falls in Schritt A eine Zustandsinformation erhalten worden ist, und
• in Abhängigkeit von der in Schritt C erhaltenen historischen Gesundheitsinformation und
• unter Verwendung einer trainierten künstlichen Intelligenz,
wobei die künstliche Intelligenz derart trainiert ist, dass sie beim Erhalt mindestens zweier Eingangsgrößen, nämlich der digitalen Umgebungsinformation oder der digitalen Zustandsinformation einerseits und der digitalen Gesundheitsinformation andererseits, ein digitales medizinisches Analyseergebnis als Ausgangsgröße ausgibt;
D) Erzeugen der wahrnehmbaren Nachricht, wobei die wahrnehmbare Nachricht derart ausgebildet ist, dass die wahrnehmbare Nachricht das medizinische Analyseergebnis für die erste menschliche Person erfassbar macht.

2. Computer-implementiertes Verfahren gemäß Anspruch 1,
wobei das Verfahren folgenden weiteren Schritt umfasst:
E) Erfassen einer Feedback-Information der ersten menschlichen Person, wobei die trainierte künstliche Intelligenz in Abhängigkeit von der Feedback-Information verändert wird;
wobei die Veränderung der künstlichen Intelligenz vorzugsweise durch einen der folgenden Schritte erfolgt:
- Nachtrainieren der künstlichen Intelligenz, zum Beispiel durch eine Feinabstimmung (*engl. fine-tuning*) oder ein Verstärkungslernen *(engl. reinforcement learning*),
- Kontext-gestützte Individualisierung der künstlichen Intelligenz, sodass einem zukünftigen Prompt eine Kontextinformation, die auf der Feedback-Information basiert, hinzugegeben wird.

3. Computer-implementiertes Verfahren gemäß einem der voranstehenden Ansprüche, wobei das Verfahren folgenden weiteren Schritt umfasst:
F) Regelmäßiges Erfassen von digitalen medizin-wissenschaftlichen Informationen, die nach einer ersten Initialisierung des Verfahrens oder einem letzten Update des Verfahrens neu erschienen sind, aus einem digitalen Repository, insbesondere aus dem Internet, zum Beispiel über einen öffentlichen Pubmed-Server;
wobei die trainierte künstliche Intelligenz in Abhängigkeit von den medizin-wissenschaftlichen Informationen verändert wird,
wobei die Veränderung der künstlichen Intelligenz vorzugsweise durch einen der folgenden Schritte erfolgt:
- Nachtrainieren der künstlichen Intelligenz, zum Beispiel durch eine überwachte Feinabstimmung (*engl. supervised fine-tuning*),
- Kontext-gestützte Individualisierung der künstlichen Intelligenz, sodass einem zukünftigen Prompt eine Kontextinformation, die auf den medizin-wissenschaftlichen Informationen basiert, hinzugegeben wird.

4. Computer-implementiertes Verfahren gemäß einem der voranstehenden Ansprüche, wobei die wahrnehmbare Nachricht in Echtzeit als Reaktion auf eine erfasste Umgebungsinformation erzeugt und der menschlichen Person wahrnehmbar zu Verfügung gestellt wird,
wobei die wahrnehmbare Nachricht bevorzugt als Audionachricht über eine Lautsprechervorrichtung, insbesondere einen Kopfhörer oder ein Kopfhörerimplantat der menschlichen Person wahrnehmbar zu Verfügung gestellt wird.

5. Computer-implementiertes Verfahren gemäß dem voranstehenden Anspruch, wobei das Verfahren folgenden weiteren Schritt umfasst:
G) Abschätzen eines Zeitraums, der durch einen Prozess, an dem die erste menschlichen Person beteiligt ist, beansprucht wird, beispielsweise einer Dauer eines Gesprächs, bei dem die erste menschliche Person beteiligt ist, in Abhängigkeit von der erfassten Umgebungsinformation;
wobei die wahrnehmbare Nachricht derart bereitgestellt wird, dass sich der Bereitstellungszeitpunkt innerhalb des in Schritt G) abgeschätzten Zeitraums befindet.

6. Computer-implementiertes Verfahren gemäß einem der voranstehenden Ansprüche,
wobei die künstliche Intelligenz ein Modell des maschinellen Lernens, vorzugsweise ein Sprachmodel, besonders bevorzugt ein multimodales Kl-Model inklusive Sprachmodell umfasst.

7. Computer-implementiertes Verfahren gemäß einem der voranstehenden Ansprüche,
wobei bei der Durchführung des Verfahrens die erste menschliche Person und die zweite menschliche Person ein und dieselbe menschliche Person sind.

8. Computer-implementiertes Verfahren gemäß dem voranstehenden Anspruch,
wobei in Schritt C) zumindest ein medizinischer Verhaltensvorschlag bestimmt wird,
wobei die wahrnehmbare Nachricht derart ausgebildet ist, dass die wahrnehmbare Nachricht den medizinischen Verhaltensvorschlag für die menschliche Person erfassbar macht und
- die wahrnehmbare Nachricht für die menschlichen Person zeitlich derart erfassbar gemacht wird, dass ein erstes Verhaltensmuster der menschlichen Person durch die Nachricht beeinflussbar ist; oder
- die wahrnehmbare Nachricht für die menschlichen Person zeitlich erfassbar gemacht wird, um einen messbaren Zustandsparameter der menschlichen Person, wie zum Beispiel die Herzfrequenz, zu regeln.

9. Computer-implementiertes Verfahren gemäß einem der Ansprüche 1 bis 6,
wobei die erste menschliche Person und die zweite menschliche Person unterschiedliche Personen sind.

10. Computer-implementiertes Verfahren gemäß einem der voranstehenden Ansprüche,
wobei die mindestens eine digitale historische Gesundheitsinformation der zweiten menschlichen Person ausgewählt ist aus einer der folgenden digitalen Informationen:
- Textdaten oder Bilddaten von schriftlichen medizinischen Befunden, insbesondere Arztbriefen oder Patientenbriefen;
- Strukturelle Daten oder Bilddaten von Blutwerten;
- Strukturelle Daten oder Bilddaten zum Impfstatus;
- Strukturelle Daten oder Bilddaten von Medikationsplänen;
- Bilddaten von bildgebenden medizinischen Untersuchungsverfahren;
- Genetische Daten;
- Daten über das Mikrobiom;
- Medizinische komplexe Daten.

11. Computer-implementiertes Verfahren gemäß einem der voranstehenden Ansprüche,
wobei in Schritt B mindestens zwei unterschiedliche Gesundheitsinformationen, vorzugsweise mindestens drei unterschiedliche Gesundheitsinformationen erhalten werden,
wobei in Schritt C das Bestimmen des digitalen medizinischen Analyseergebnisses in Abhängigkeit von den mindestens zwei in Schritt B erhaltenen Gesundheitsinformationen, vorzugsweise in Abhängigkeit von den mindestens drei in Schritt B erhaltenen Gesundheitsinformationen, erfolgt.

12. Computer-implementiertes Verfahren gemäß einem der voranstehenden Ansprüche,
wobei die erste Computerdatenbank innerhalb einer ersten Serverumgebung angeordnet ist,
wobei die trainierte künstliche Intelligenz innerhalb einer zweiten, sich von der ersten Serverumgebung unterscheidenden Serverumgebung angeordnet ist,
sodass die trainierte künstliche Intelligenz derart abgeschirmt ist, dass die trainierte künstliche Intelligenz nicht unmittelbar auf die in der ersten Computerdatenbank abgelegten historischen Gesundheitsinformationen zugreifen kann,
wobei die trainierte künstliche Intelligenz vorzugsweise derart abgeschirmt ist, dass die trainierte künstliche Intelligenz nicht auf das Internet zugreifen kann

13. Computer-implementiertes Verfahren gemäß dem voranstehenden Anspruch,
wobei Gesundheitsinformationen, die zum Zwecke der Verarbeitung durch die künstliche Intelligenz in die zweite Serverumgebung übertragen werden, ausschließlich temporär in der zweiten Serverumgebung gespeichert werden, um von der künstlichen Intelligenz als Eingangsgrößen verwendet zu werden,
wobei die Gesundheitsinformationen vorzugsweise ausschließlich quasi-anonymisiert in der zweiten Serverumgebung gespeichert werden, um von der künstlichen Intelligenz als Eingangsgrößen verwendet zu werden.

14. Computer-implementiertes Verfahren gemäß einem der voranstehenden Ansprüche,
wobei in Schritt C ein Therapievorschlag bestimmt wird,
wobei das Verfahren folgenden weiteren Schritt umfasst:
Simulieren einer körperlichen Reaktion der zweiten menschlichen Person auf den in Schritt C bestimmten Therapievorschlag in Abhängigkeit von zumindest einer erhaltenen Gesundheitsinformation der zweiten menschlichen Person, sodass ein Simulationsergebnis eine Erfolgswahrscheinlichkeit des Therapievorschlags umfasst,
wobei der in Schritt C bestimmte Therapievorschlag in Abhängigkeit von dem Simulationsergebnis verändert oder beibehalten wird, bevor die Nachricht in Schritt D in Abhängigkeit von dem Therapievorschlag erzeugt.

15. Computer-implementiertes Verfahren zum Spezifizieren einer künstlichen Intelligenz, wobei die künstliche Intelligenz mindestens ein trainiertes oder teiltrainiertes Foundation-Modell (z.B. ein Large Language Model oder ein multimodales Modell) umfasst, wobei das Foundation-Modell Text, Bild, Audio, genetische Sequenzen oder andere Datenformate verarbeiten kann,
wobei der künstlichen Intelligenzin einem ersten Spezifizierungsschritt mit der Methode "Retrieval-Augmented Generation" spezifisches Wissen angeeignet wird,
wobei das Foundation-Modell im ersten Spezifizierungsschritt Textinformationen aus einer ersten Datenquelle und Textinformationen aus einer zweiten Datenquelle erhält, wobei das Foundation-Modell im ersten Spezifizierungsschritt zusätzlich eine Priorisierungsinformation bezüglich der ersten Datenquelle und der zweiten Datenquelle erhält, sodass das Foundation-Modell Textinformationen aus der ersten Datenquelle stärker gewichtet als Textinformationen aus der zweiten Datenquelle,
wobei die erste Datenquelle und die zweite Datenquelle derart ausgewählt sind, dass sie medizinische, insbesondere humanmedizinische Fachinformationen, enthalten,
wobei die erste Datenquelle vorzugsweise medizinische Leitlinien von medizinischen Fachgesellschaften umfasst.

## Geänderte Patentansprüche

### Geänderte Patentansprüche gemäss Regel 137(2) EPÜ.

1. Computer-implementiertes Arbeitsverfahren zum Erzeugen einer mit menschlichen Sinnesorgangen wahrnehmbaren Nachricht für eine erste menschliche Person, wobei das Verfahren folgende Schritte umfasst:
A) Dauerhaftes oder wiederholtes Erhalten von mindestens einer zeitlich veränderlichen und mit einem Mobilgerät messbaren digitalen Umgebungsinformation der ersten menschlichen Person, nämlich einer digitalen akustischen Information in Form einer digitalen Sprach- und/oder Tonaufnahme eines Mikrofons;
B) Erhalten von mindestens einer digitalen historischen Gesundheitsinformation einer zweiten menschlichen Person aus einer ersten Computerdatenbank;
C) Bestimmen eines digitalen medizinischen Analyseergebnisses
• in Abhängigkeit von der erhaltenen Umgebungsinformation und
• in Abhängigkeit von der erhaltenen historischen Gesundheitsinformation und
• unter Verwendung einer trainierten künstlichen Intelligenz,
wobei die künstliche Intelligenz derart trainiert ist, dass sie beim Erhalt mindestens zweier Eingangsgrößen, nämlich der digitalen Umgebungsinformation einerseits und der digitalen Gesundheitsinformation andererseits, ein digitales medizinisches Analyseergebnis als Ausgangsgröße ausgibt;
D) Erzeugen der wahrnehmbaren Nachricht, wobei die wahrnehmbare Nachricht derart ausgebildet ist, dass die wahrnehmbare Nachricht das medizinische Analyseergebnis für die erste menschliche Person erfassbar macht;
wobei das Verfahren folgenden weiteren Schritt umfasst:
G) Abschätzen einer Dauer eines Gesprächs, bei dem die erste menschliche Person beteiligt ist, in Abhängigkeit von der erfassten Umgebungsinformation;
wobei die wahrnehmbare Nachricht derart bereitgestellt wird, dass sich der Bereitstellungszeitpunkt innerhalb der in Schritt G) abgeschätzten Dauer befindet,
wobei die künstliche Intelligenz ein Modell des maschinellen Lernens umfasst,
wobei die erste menschliche Person und die zweite menschliche Person unterschiedliche Personen sind.

2. Computer-implementiertes Verfahren gemäß Anspruch 1,
wobei das Verfahren folgenden weiteren Schritt umfasst:
E) Erfassen einer Feedback-Information der ersten menschlichen Person,
wobei die trainierte künstliche Intelligenz in Abhängigkeit von der Feedback-Information verändert wird;
wobei die Veränderung der künstlichen Intelligenz vorzugsweise durch einen der folgenden Schritte erfolgt:
- Nachtrainieren der künstlichen Intelligenz, zum Beispiel durch eine Feinabstimmung (*engl. fine-tuning*) oder ein Verstärkungslernen *(engl. reinforcement learning*),
- Kontext-gestützte Individualisierung der künstlichen Intelligenz, sodass einem zukünftigen Prompt eine Kontextinformation, die auf der Feedback-Information basiert, hinzugegeben wird.

3. Computer-implementiertes Verfahren gemäß einem der voranstehenden Ansprüche, wobei das Verfahren folgenden weiteren Schritt umfasst:
F) Regelmäßiges Erfassen von digitalen medizin-wissenschaftlichen Informationen, die nach einer ersten Initialisierung des Verfahrens oder einem letzten Update des Verfahrens neu erschienen sind, aus einem digitalen Repository, insbesondere aus dem Internet, zum Beispiel über einen öffentlichen Pubmed-Server;
wobei die trainierte künstliche Intelligenz in Abhängigkeit von den medizin-wissenschaftlichen Informationen verändert wird,
wobei die Veränderung der künstlichen Intelligenz vorzugsweise durch einen der folgenden Schritte erfolgt:
- Nachtrainieren der künstlichen Intelligenz, zum Beispiel durch eine überwachte Feinabstimmung (*engl. supervised fine-tuning*),
- Kontext-gestützte Individualisierung der künstlichen Intelligenz, sodass einem zukünftigen Prompt eine Kontextinformation, die auf den medizin-wissenschaftlichen Informationen basiert, hinzugegeben wird.

4. Computer-implementiertes Verfahren gemäß einem der voranstehenden Ansprüche, wobei die wahrnehmbare Nachricht in Echtzeit als Reaktion auf eine erfasste Umgebungsinformation erzeugt und der menschlichen Person wahrnehmbar zu Verfügung gestellt wird,
wobei die wahrnehmbare Nachricht bevorzugt als Audionachricht über eine Lautsprechervorrichtung, insbesondere einen Kopfhörer oder ein Kopfhörerimplantat der menschlichen Person wahrnehmbar zu Verfügung gestellt wird.

5. Computer-implementiertes Verfahren gemäß einem der voranstehenden Ansprüche, wobei die künstliche Intelligenz ein Sprachmodel, besonders bevorzugt ein multimodales KI-Model inklusive Sprachmodell umfasst.

6. Computer-implementiertes Verfahren gemäß einem der voranstehenden Ansprüche, wobei die mindestens eine digitale historische Gesundheitsinformation der zweiten menschlichen Person ausgewählt ist aus einer der folgenden digitalen Informationen:
- Textdaten oder Bilddaten von schriftlichen medizinischen Befunden, insbesondere Arztbriefen oder Patientenbriefen;
- Strukturelle Daten oder Bilddaten von Blutwerten;
- Strukturelle Daten oder Bilddaten zum Impfstatus;
- Strukturelle Daten oder Bilddaten von Medikationsplänen;
- Bilddaten von bildgebenden medizinischen Untersuchungsverfahren;
- Genetische Daten;
- Daten über das Mikrobiom;.

7. Computer-implementiertes Verfahren gemäß einem der voranstehenden Ansprüche, wobei in Schritt B mindestens zwei unterschiedliche Gesundheitsinformationen, vorzugsweise mindestens drei unterschiedliche Gesundheitsinformationen erhalten werden,
wobei in Schritt C das Bestimmen des digitalen medizinischen Analyseergebnisses in Abhängigkeit von den mindestens zwei in Schritt B erhaltenen Gesundheitsinformationen, vorzugsweise in Abhängigkeit von den mindestens drei in Schritt B erhaltenen Gesundheitsinformationen, erfolgt.

8. Computer-implementiertes Verfahren gemäß einem der voranstehenden Ansprüche, wobei die erste Computerdatenbank innerhalb einer ersten Serverumgebung angeordnet ist,
wobei die trainierte künstliche Intelligenz innerhalb einer zweiten, sich von der ersten Serverumgebung unterscheidenden Serverumgebung angeordnet ist,
sodass die trainierte künstliche Intelligenz derart abgeschirmt ist, dass die trainierte künstliche Intelligenz nicht unmittelbar auf die in der ersten Computerdatenbank abgelegten historischen Gesundheitsinformationen zugreifen kann,
wobei die trainierte künstliche Intelligenz vorzugsweise derart abgeschirmt ist, dass die trainierte künstliche Intelligenz nicht auf das Internet zugreifen kann

9. Computer-implementiertes Verfahren gemäß dem voranstehenden Anspruch,
wobei Gesundheitsinformationen, die zum Zwecke der Verarbeitung durch die künstliche Intelligenz in die zweite Serverumgebung übertragen werden, ausschließlich temporär in der zweiten Serverumgebung gespeichert werden, um von der künstlichen Intelligenz als Eingangsgrößen verwendet zu werden,
wobei die Gesundheitsinformationen vorzugsweise ausschließlich quasi-anonymisiert in der zweiten Serverumgebung gespeichert werden, um von der künstlichen Intelligenz als Eingangsgrößen verwendet zu werden.

10. Computer-implementiertes Verfahren gemäß einem der voranstehenden Ansprüche, wobei in Schritt C ein Therapievorschlag bestimmt wird,
wobei das Verfahren folgenden weiteren Schritt umfasst:
Simulieren einer körperlichen Reaktion der zweiten menschlichen Person auf den in Schritt C bestimmten Therapievorschlag in Abhängigkeit von zumindest einer erhaltenen Gesundheitsinformation der zweiten menschlichen Person, sodass ein Simulationsergebnis eine Erfolgswahrscheinlichkeit des Therapievorschlags umfasst,
wobei der in Schritt C bestimmte Therapievorschlag in Abhängigkeit von dem Simulationsergebnis verändert oder beibehalten wird, bevor die Nachricht in Schritt D in Abhängigkeit von dem Therapievorschlag erzeugt.

11. Computerprogammprodukt, wobei das Computerprogrammprodukt Befehle umfasst, die bei der Ausführung des Programms durch einen Computer diesen veranlassen, eines der Verfahren gemäß der voranstehenden Ansprüche auszuführen.

12. Computerlesbares Speichermedium, umfassend Befehle, die bei der Ausführung durch einen Computer diesen veranlassen, eines der Verfahren gemäß Ansprüchen 1 bis 10 auszuführen.
